# EUROPÄISCHE PATENTSCHRIFT

(11) **EP 2 621 479 B1**
(45) Veröffentlichungstag und Bekanntmachung des Hinweises auf die Patenterteilung: **29.11.2017**
(21) Anmeldenummer: 11760489.2
(22) Anmeldetag: 26.09.2011
(51) Int. Cl.: A61K 9/50, A61K 9/00

(54) **PROTEKTIVE ÜBERZÜGE FÜR SAURE WIRKSTOFFE**
PROTECTIVE COATING FOR ACIDIC DRUGS
REVETMENT PROTECTEUR D'UN MEDICAMENT ACIDE

(30) Priorität: 27.09.2010 EP 10179816
(43) Veröffentlichungstag der Anmeldung: 07.08.2013
(73) Patentinhaber: BASF SE, 67056 Ludwigshafen am Rhein (DE)
(72) Erfinder: KOLTER, Karl, 67117 Limburgerhof (DE); ANGEL, Maximilian, 67105 Schifferstadt (DE)
(74) Vertreter: BASF IP Association
(86) Internationale Anmeldenummer: PCT/EP2011/066627
(87) Internationale Veröffentlichungsnummer: WO 2012/041788

(56) Entgegenhaltungen:
- EP-A2- 1 110 544
- WO-A1-93/24109
- WO-A1-03/075896
- WO-A1-2008/080774
- WO-A2-01/80826
- WO-A2-2010/139654

## Beschreibung

Die vorliegende Erfindung betrifft protektive Überzüge für pharmazeutische Dosierungsformen mit sauren Wirkstoffen, die zwecks Geschmacksmaskierung oder zum Schutz gegen Feuchtigkeit mit einem Filmüberzug auf Basis eines kationischen Polymers, welches mittels radikalischer Emulsionspolymerisation eines N,N-Diethylaminoethylmethacrylat enthaltenden Monomergemischs erhalten wird, versehen werden, wobei die Überzüge mindestens aus einer inneren Schicht und einer äusseren Schicht bestehen und die innere Überzugsschicht aus einem neutralen wasserlöslichen Polymer und optional Pigmenten und weiteren pharmazeutisch üblichen Hilfsstoffen besteht und als neutrale Polymere der inneren Überzugsschicht Polyvinylalkohole, Polyalkylenglykol - Polyvi-nylalkohol-Pfropfcopolymere, Polyvinylpyrrolidone, Vinylpyrrolidon - Vinylacetat-Copolymere, alkylierte und hydroxyalkylierte Cellulosen oder Stärken oder Gemische solcher Polymere eingesetzt werden.

Die DE-AS 1090381 beschreibt ein Verfahren zum Überziehen von Arzneiformen mit im Magen löslichen Dragiermassen. Diese enthalten ein Copolymer aus 20 bis 80 % wenigstens eines Aminoesters der (Meth)acrylsäure und 80 bis 20 % eines Monomers, das als Homopolymer ein wasserunlösliches Polymerisat bildet. Als konkrete Beispiele für geeignete polymerisierbare Aminoester werden die Ester der Acrylsäure und (Meth)acrylsäure mit N,N-Dimethylaminoethanol, N,N-Diethylaminoethanol, N,N-Dimethylaminopropanol und N-(hydroxyethyl)morpholin genannt. Als geeignete Comonomere werden niedere Ester der Acrylsäure und vorzugsweise der (Meth)acrylsäure, wie Acrylsäureethylester, (Meth)acrylsäuremethyl-, -butyl- und -hexylester genannt. Die Herstellung erfolgt durch Lösungspolymerisation in einem organischen Lösungsmittel; ein Ausführungsbeispiel ist nicht angegeben.

Die DE-AS 1219175 beschreibt ein Verfahren zur Herstellung von veterinärmedizinischen Wirkstoffzubereitungen, die gegen die Einwirkung von Pansensäften von Wiederkäuern geschützt sind. Dazu werden diese Zubereitungen mit Copolymeren überzogen, die N,N-Dialkylaminoalkyl(meth)acrylamide und ein Comonomer einpolymerisiert enthalten, das ausgewählt ist unter (Meth)acrylaten, Acrylnitril und Vinylaromaten. Copolymere auf Basis von N,N-Dialkylaminoalkyl(meth)acrylaten werden nach der Lehre dieses Dokuments als nachteilig angesehen, da die Estergruppe im Vergleich zur Amidgruppe im basischen Milieu eher verseift.

Die DE-OS 2135073 beschreibt Überzugsmittel für Arzneiformen, die eine wässrige Polymerdispersion enthalten, wobei das Polymer zu 10 bis 55 Gew.-% aus Monomeren mit einer Carboxylgruppe und/oder einer Monoalkyl- oder Dialkylaminoalkylestergruppe aufgebaut ist. Als geeignetes Monomer wird neben einer Vielzahl weiterer auch Diethylaminoethylmethacrylat (DEAEMA) genannt. Als geeignete Comonomere werden die niederen Ester der (Meth)acrylsäure, vorzugsweise Methylmethacrylat, (Meth)acrylnitril, Vinylaromaten, Vinylchlorid und Vinylacetat genannt. Die Herstellung erfolgt durch wässrige Emulsionspolymerisation, vorzugsweise nach dem Emulsionszulaufverfahren. Konkrete Emulsionspolymerisate auf Basis von DEAEMA sind nicht offenbart.

Die DE-AS 2512238 lehrt zur Bereitstellung von Bindemitteln für Arzneimittelüberzüge mit geringem Restmonomerengehalt die Verwendung eines durch Sprühtrocknen einer Polymerdispersion erhaltenen Pulvers zur Herstellung von Überzugslösungen für diese Arzneiformen. Bezüglich der zur Sprühtrocknung eingesetzten Dispersionen wird auf die DE 1090381, DE 1219175 und DE 2135073 Bezug genommen.

Die DE-OS 2838278 beschreibt Beschichtungen für orale Darreichungsformen für Wiederkäuer aus a) mindestens einem filmbildenden Polymer mit mindestens einer basischen Aminogruppe und mit einem Stickstoffgehalt von 3 bis 14 %, das in wässrigem Pansenmedium bei einem pH-Wert von über 5,5 innerhalb von 24 Stunden löslich ist, und b) mindestens einer hydrophoben, in dem Polymer dispergierten Substanz, die ausgewählt ist unter C₁₂-C₃₂-Fettsäuren, Al-Salzen dieser Fettsäuren und/oder Polycarbonsäuren.

Zur Herstellung der Beschichtung wird eine Lösung in einem organischen Lösungsmittel eingesetzt. Als geeignete Polymere wird eine Vielzahl stickstoffhaltiger Homo- und Copolymere aufgeführt, ohne auf geeignete Verfahren zu deren Herstellung einzugehen. Als Ausführungsbeispiel 29 wird dabei ein Copolymer aus 40 % N,N-Diethylaminoethylmethacrylat aufgeführt, ohne jedoch ein Verfahren zu seiner Herstellung anzugeben.

Die GB 1324087 beschreibt Überzugspolymere für orale Darreichungsformen für Wiederkäuer, die a)wenigstens ein N,N-Dialkylaminoalkyl(meth)acrylat und b) wenigstens eine ethylenisch ungesättigte Verbindung, die ausgewählt ist unter Vinylaromaten und deren Derivaten, Vinylestern, Estern der (Meth)acrylsäure und Acrylnitril einpolymerisiert enthalten. Als geeignete Monomere a) werden N,N-Dimethylaminoethylmethacrylat (DMAEMA) und tert.-Butylaminoethylmethacrylat (TBAEMA) offenbart. Als Comonomer b) wird insbesondere Methylmethacrylat als ungeeignet angesehen, da es zur Bildung von zu brüchigen Überzügen neigt. Als geeignete Polymerisationsverfahren werden Substanz-, Suspensions-, Lösungs- und Emulsionspolymerisation angegeben. Die Copolymere der Ausführungsbeispiele wurden durch Lösungspolymerisation hergestellt.

Die DE 3426587 A1 beschreibt ein Verfahren zum Überziehen von Arzneiformen durch Aufbringen eines Films aus einem flüssigen, filmbildenden Überzugsmittel, welches ein gelöstes Polymerisat mit seitenständigen tertiären Ammoniumsalzgruppen enthält. Zur Herstellung dieser Polymerlösungen können unter anderem Copolymere auf Basis von N,N-Dialkylaminoalkyl(meth)acrylaten mit wässrigen anorganischen oder organischen Säuren in wässrige Ammoniumsalzlösungen überführt werden.

Die DE 3049179 A1 ist eine Zusatzanmeldung zur DE 2512238 und betrifft die Verwendung eines durch Sprühtrocknen nach der Lehre des letztgenannten Dokuments gewonnenen Pulvers in Form einer wässrigen Suspension, die zusätzlich ein Weichmachungsmittel enthält, zur Herstellung von Überzügen durch Thermogelierung.

Die EP 0058765 A2 beschreibt in Magensaft lösliche oder quellbare Überzugsmassen für Arzneiformen, die als Bindemittel ein Emulsionspolymerisat auf Basis von N,N-Dialkylaminoalkyl(meth)acrylaten enthalten, wobei zwischen der Aminogruppe und der (Meth)acrylatgruppe sich eine verzweigte Alkylen- oder Aralkylengruppe mit wenigstens drei in gerader Kette angeordneten Kohlenstoffatomen befindet.

Die WO 2005/055986 und WO 2005/056619 beschreiben Polymere mit vom pH-Wert abhängigem Quell-/Lösungsverhalten und deren Einsatz in Arzneiformen.

Die WO 00/05307 beschäftigt sich mit der Bereitstellung von Überzugs- und Bindemitteln für Arzneiformen, die (Meth)acrylat-Copolymere enthalten, die Monomerreste mit tertiären Aminogruppen aufweisen, wobei eine einfache trockene oder wässrige Weiterverarbeitung möglich sein soll. Dazu lehrt dieses Dokument ein Verfahren, bei dem man (a) ein Copolymer aus C₁-C₄-Estern der (Meth)acrylsäure und (Meth)acrylat-Monomeren, die tertiäre Ammoniumgruppen aufweisen, (b) einen Weichmacher und (c) einen Emulgator mit einem HLB-Wert von mindestens 14 miteinander vermengt und daraus das Überzugs- oder Bindemittel durch Schmelzen, Gießen, Ausstreichen oder Aufsprühen herstellt, wobei das Copolymer (a) in Pulverform mit einer mittleren Teilchengröße von 1 bis 40 µm eingebracht wird. Die dabei erzielte Verarbeitbarkeit wird der Bereitstellung des Copolymeren (a) in Pulverform mit extrem geringer Korngröße zugeschrieben.

Die WO 02/067906 betrifft Überzugs- und Bindemittel mit verbesserter Wasserdampfdurchlässigkeit gegenüber den in der WO 00/05307 beschriebenen. Dabei erfolgt die Herstellung der Überzugs- und Bindemittel mit einer Mischung, die (a) ein Copolymer aus C₁-C₄-Estern der (Meth)acrylsäure und weitere (Meth)acrylat-Monomere mit funktionellen tertiären Ammoniumgruppen in Pulverform mit einer mittleren Teilchengröße von 1 bis 40 µm, (b) einen Emulgator mit einem HLB-Wert von mindestens 14 und (c) eine C₁₂-C₁₈-Monocarbonsäure oder eine C₁₂-C₁₈-Hydroxylverbindung enthält.

Die WO 2004/019918 beschreibt Überzugs- und Bindemittel, die bezüglich ihrer Zusammensetzung den in der WO 00/05307 und WO 02/067906 beschriebenen entsprechen.

Gemäß der US 6,696,085 B2 soll ein Methacrylsäure-Copolymer Typ C als Zerfallsmittel eingesetzt werden. Das Methacrylsäure-Copolymer Typ C ist ein magensaft-resistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

Die Matrixkomponenten auf Basis von Zuckeralkoholen, Sprengmitteln und unlöslichen Polymeren sind allgemein für pharmazeutische Anwendungen aus der WO 2007/071581 bekannt.

Aus der WO 2009/016258 ist die Herstellung der wässrigen Polymerdispersionen kationischer Polymere auf Basis von N,N-Diethylaminoethylmethacrylat wie sie erfindungsgemäß zum Einsatz kommen und deren Verwendung zum Überziehen von Arzneistoffen bekannt. In dieser Schrift wird auch die Applikation der genannten Polymere auf Ibuprofenpellets beschrieben. Es hat sich jedoch herausgestellt, dass diese Zubereitungen nicht lagerstabil sind, bei Lagerung verkleben, die Geschmacksmaskierung nachlässt und sich die Freisetzung massiv verändert.

Die WO 03/075896 A1 betrifft Arzneiformen mit verzögerter Freisetzung, wobei der die Freisetzung steuernde Überzug zwingend als filmbildende Komponente das bei allen pH-Werten wasserunlösliche Polyvinylacetat (PVAc)enthält, wobei das zugrundeliegende Problemdarin besteht, Tabletten mit verzögerter Freisetzung so zu überziehen, dass die Tabletten gegen mechanischen Stress geschützt sind und kein unter Umständen lebensbedrohendes "Dose Dumping" auftreten kann. Zusätzlich zu diesem PVAc-haltigen Überzug kann eine weitere Überzugsschicht unter oder der PVAc-haltigen Schicht aufgebracht werden, wobei die zusätzliche Schicht bevorzugt ein enterisches Überzugsmittel enthält. Damit ist der Schichtenaufbau ein ganz anderer, weil eine hydrophobe PVAc-haltige Schicht mit einer enterischen Schicht kombiniert wird.

Die WO 93/24109 A1 beschreibt geschmacksmaskierte Granulate für schnell freisetzende Arzneiformen von Cimetidin, die zur Geschmacksmaskierung mit zwei Überzügen, die beide jeweils Trimethylammoniumethylmethacrylatchlorid (TAMCI) als Comonomer enthalten, wobei die Freisetzung über den Anteil an TAMCI gesteuert wird. Cimetidin ist kein saurer Wirkstoff.

Die EP 1 110 544 A2 beschreibt die Verwendung von PVAc-haltigen Überzügen zur Geschmacksmaskierung. PVAc ist wasserunlöslich und chemisch völlig verschieden von den erfindungsgemäß zur Geschmacksmaskierung verwendeten DEAEMA-Copolymeren.

Die WO 2008/080774 A1 beschreibt die Herstellung von pigmentierten Überzugsmitteln auf Basis von Polyvinylalkohol-Polyethylenglykol-Pfropfcopolymeren durch Extrusion und die Anwendung entsprechender Überzugsmittel.

Die WO 01/80826 A2 beschreibt geschmacksmaskierende Überzüge für im Magen schnell freisetzende Arzneiformen. Die Überzugsschicht enthält eine Kombination von Dimethylaminoethyl-haltigen Copolymeren mit Celluloseestern und zwingend einem alkalischen "Modifier", wobei der alkalische Modifier dazu bestimmt ist, zum einen die Freisetzung zu steuern, zum anderen aber als Puffersubstanz zur Stabilisierung des Überzugs.

Der vorliegenden Erfindung liegt die Aufgabe zu Grunde, verbesserte Filmüberzugsmittel für pharmazeutische Darreichungsformen, enthaltend saure Wirkstoffe, die auch bei längerer oder temperaturbelastender Lagerung keine Veränderung der Geschmacksmaskierung, des Schutzes vor Feuchte und im Freisetzungsverhalten aufweisen, bereitzustellen, die die Nachteile des Standes der Technik nicht aufweisen.

Demgemäß wurden protektive Überzüge und mit solchen Überzügen versehenen Darreichungsformen gefunden, in denen ein mindestens einen sauren Wirkstoff enthaltender Kern mit mindestens einer inneren und einer äußeren Überzugsschicht versehen ist, wobei die äussere Schicht ein kationisches Polymer, welches ein Emulsionspolymerisat aus N,N-Diethylaminoethylmethacrylat und weiteren Monomeren darstellt, enthält, und die innere Überzugsschicht aus einem neutralen wasserlöslichen Polymer und optional Pigmenten und weiteren pharmazeutisch üblichen Hilfsstoffen besteht und als neutrale Polymere der inneren Überzugsschicht Polyvinylalkohole, Polyalkylenglykol - Polyvinylalkohol-Pfropfcopolymere, Polyvinylpyrrolidone, Vinylpyrrolidon - Vinylacetat-Copolymere, alkylierte und hydroxyalkylierte Cellulosen oder Stärken oder Gemische solcher Polymere eingesetzt werden.

Als saure Wirkstoffe im Sinne der Erfindung werden Wirkstoffe mit freien Carboxyl-, Sulfonsäure oder Phosphonsäuregruppen, aciden Hydroxylgruppen, aciden N-H Gruppen oder aciden C-H Gruppen verstanden. Der pks-Wert dieser Wirkstoffe liegt zwischen 6,5 und 0,5, bevorzugt zwischen 5,0 und 1,0. Der pKs-Wert bezeichnet den negativen dekadischen Logarithmus der Säurekonstante. Die Säurekonstante wird üblicherweise durch Titration mit Lauge ermittelt.

Der erfindungsgemäße Überzug weist einen mindestens zweischichtigen Aufbau auf, mit einer inneren Schicht bestehend aus einem neutralen wasserlöslichen Polymer und optional Pigmenten und weiteren pharmazeutisch üblichen Hilfsstoffen. Unter einem neutralen wasserlöslichen Polymer ist ein Polymer ohne saure oder basische Gruppe zu verstehen. Wasserlöslich bedeutet, dass bei 25 °C mindestens 50 g in 1 L Wasser löslich sind.

Die äußere Schicht enthält
i) als Komponente A ein durch radikalische Polymerisation erhaltenes Polymer aus
   a) N,N-Diethylaminoethylmethacrylat, und
   b) wenigstens eine radikalisch polymerisierbare Verbindung, ausgewählt unter Estern α,β-ethylenisch ungesättigter Mono- und Dicarbonsäuren mit C₁-C₈-Alkanolen,
ii) optional als Komponente B ein oder mehrere Antioxidantien,
iii) optional als Komponente C einen oder mehrere Weichmacher,
iv) optional als Komponenten D weitere pharmazeutische Hilfsstoffe

Als wasserlösliche Polymere der inneren Schicht werden eingesetzt:
Polyvinylalkohole, Polyalkylenglykol - Polyvinylalkohol-Pfropfcopolymere, Polyvinylpyrrolidone, Vinylpyrrolidon - Vinylacetat-Copolymere, alkylierte und hydroxyalkylierte Cellulosen oder Stärken wie z. B. Hydroxypropylcellulose, Hydroxypropylmethylcellulose, Hydroxyethylcellulose, Methylcellulose, Hydroxyethylstärke.

Die Schichtdicke der inneren Schicht kann 1 bis 50 µm betragen, vorzugsweise 2 bis 25 µm und besonders bevorzugt 5 bis 15 µm.
Die Schichtdicke der äußeren Schicht kann 5 bis 200 µm, vorzugsweise 10 bis 150 µm und besonders bevorzugt 20 bis 100 µm betragen.

Die verwendeten Überzugsmittel für die äussere Schicht basieren auf wässrigen Polymerdispersionen, welche durch radikalische Emulsionspolymerisation eines Monomergemischs M), enthaltend
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
in einem wässrigen Medium bei einem pH-Wert von wenigstens 8, erhalten werden.

Die Überzugsmittel in Form von wässrigen Polymerdispersionen enthalten vorzugsweise keine zusätzlichen organischen Lösungsmittel.

Erfindungsgemäß dienen die Überzugsmittel zur Herstellung von pharmazeutischen Dosierungsformen, die im sauren Milieu des Magens schnell freisetzend sein sollen. D.h. die erfindungsgemäßen Überzüge sind magensaftlöslich. Schnell freisetzend bedeutet in diesem Zusammenhang, dass nach 60 min mindestens 80 % des Wirkstoffs freigesetzt sind. Erfindungsgemäß erhaltene Überzüge sollen sich in Mundhöhle und Rachen im neutralen oder nahezu neutralen Milieu des Speichels nicht auflösen.
Die erfindungsgemäßen Überzugsmittel können zur Geschmacksmaskierung oder zum Schutz vor Feuchtigkeit verwendet werden. Die Wasserdampfpermeabilität der Überzüge ist sehr niedrig, wodurch feuchtigkeitsempfindliche Wirkstoffe geschützt werden.

### Komponente A

### Monomer a)

Als Monomer a) wird erfindungsgemäß N,N-Diethylaminoethylmethacrylat eingesetzt.

Zur Herstellung der erfindungsgemäßen wässrigen Polymerdispersionen Pd) wird die Komponente a) vorzugsweise in einer Menge von 25 bis 65 Gew.-%, besonders bevorzugt 30 bis 60 Gew.-%, insbesondere 38 bis 48 Gew.-%, speziell 43 bis 47 Gew.-%, bezogen auf das Gesamtgewicht der zur Polymerisation eingesetzten Monomere, eingesetzt.

### Monomer b)

Die Komponente b) ist ausgewählt unter Estern alpha, beta-ethylenisch ungesättigter Mono-und Dicarbonsäuren mit C₁-C₈-Alkanolen.

Geeignete Verbindungen b) sind Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, sec.-Butyl(meth)acrylat, tert.-Butyl(meth)acrylat, tert.-Butylethacrylat, n-Hexyl(meth)acrylat, n-Heptyl(meth)acrylat, n-Octyl(meth)acrylat, 1,1,3,3-Tetramethylbutyl(meth)acrylat und Ethylhexyl(meth)acrylat.

Besonders bevorzugt wird als Komponente b) Methylmethacrylat oder ein Methylmethacrylat enthaltendes Monomergemisch eingesetzt.

Zur Herstellung der erfindungsgemäßen wässrigen Polymerdispersionen wird die Komponente b) vorzugsweise in einer Menge von 35 bis 75 Gew.- %, besonders bevorzugt 40 bis 70 Gew.-%, insbesondere 52 bis 62 Gew.-%, speziell 53 bis 57 Gew.-%, bezogen auf das Gesamtgewicht der zur Polymerisation eingesetzten Monomere, eingesetzt.

### Monomer c)

Die zur Herstellung der Polymerdispersionen eingesetzten Monomergemische M) können zusätzlich wenigstens ein weiteres Monomer c) enthalten. Die zusätzlichen Monomere c) sind vorzugsweise ausgewählt unter Estern alpha, beta-ethylenisch ungesättigter Mono- und Dicarbonsäuren mit C₉-C₃₀-Alkanolen und C₂-C₃₀-Alkandiolen, Amiden alpha, beta-ethylenisch ungesättigter Mono- und Dicarbonsäuren mit C₂-C₃₀-Aminoalkoholen, die eine primäre oder sekundäre Aminogruppe aufweisen, primären Amiden alpha, beta-ethylenisch ungesättigter Monocarbonsäuren und deren N-Alkyl- und N,N-Dialkylderivaten, N-Vinyllactamen, offenkettigen N-Vinylamidverbindungen, Estern von Vinylalkohol und Allylalkohol mit C₁-C₃₀-Monocarbonsäuren, Vinylethern, Vinylaromaten, Vinylhalogeniden, Vinylidenhalogeniden, C₂-C₈-Monoolefinen, ungesättigte Nitrile, nicht aromatischen Kohlenwasserstoffen mit mindestens zwei konjugierten Doppelbindungen und Mischungen davon.

Geeignete zusätzliche Monomere c) sind Ester alpha, beta-ethylenisch ungesättigter Mono- und Dicarbonsäuren mit C₉-C₃₀-Alkanolen, wie n-Nonyl(meth)acrylat, n-Decyl(meth)acrylat, n-Undecyl(meth)acrylat, Tridecyl(meth)acrylat,
Myristyl(meth)acrylat, Pentadecyl(meth)acrylat, Palmityl(meth)acrylat,
Heptadecyl(meth)acrylat, Nonadecyl(meth)acrylat, Arachinyl(meth)acrylat, Behenyl(meth)acrylat, Lignoceryl(meth)acrylat, Cerotinyl(meth)acrylat,
Melissinyl(meth)acrylat, Palmitoleinyl(meth)acrylat, Oleyl(meth)acrylat,
Linolyl(meth)acrylat, Linolenyl(meth)acrylat, Stearyl(meth)acrylat,
Lauryl(meth)acrylat und Mischungen davon.

Geeignete zusätzliche Monomere c) sind weiterhin Ester alpha, beta-ethylenisch ungesättigter Mono- und Dicarbonsäuren mit C₂-C₃₀-Alkandiolen, wie 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc.

Geeignete zusätzliche Monomere c) sind weiterhin primäre Amide alpha, beta-ethylenisch ungesättigter Monocarbonsäuren und deren N-Alkyl- und N,N-Dialkylderivate, wie Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N-(n-Octyl)(meth)acrylamid, N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arachinyl(meth)acrylamid, N-Behenyl(meth)acrylamid, N-Lignoceryl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid, N,N-Dimethyl(meth)acrylamid, N,N-Diethyl(meth)acrylamid, Morpholinyl(meth)acrylamid.

Als zusätzliche Monomere c) eignen sich weiterhin N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und N-Vinylcaprolactam eingesetzt.

Als Monomere c) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.

Geeignete zusätzliche Monomere c) sind weiterhin Vinylacetat, Vinylpropionat, Vinylbutyrat und Mischungen davon.

Geeignete zusätzliche Monomere c) sind weiterhin Ethylen, Propylen, Isobutylen, Butadien, Styrol, alpha-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere c) können einzeln oder in Form von beliebigen Mischungen eingesetzt werden.
Zur Herstellung der erfindungsgemäßen wässrigen Polymerdispersionen wird die Komponente c) vorzugsweise in einer Menge von 0 bis 80 Gew.-%, bezogen auf das Gesamtgewicht der zur Polymerisation eingesetzten Monomere, eingesetzt. Eine spezielle Ausführungsform betrifft Polymerdispersionen Pd), die kein zusätzliches Monomer c) einpolymerisiert enthalten. Soweit vorhanden, wird die Komponente c) vorzugsweise in einer Menge von 0,1 bis 70 Gew.-%, besonders bevorzugt 1 bis 60 Gew.-%, insbesondere 5 bis 50 Gew.-%, bezogen auf das Gesamtgewicht der zur Polymerisation eingesetzten Monomere, eingesetzt.

Vorzugsweise wird kein Monomer c) verwendet.

### Monomer d)

Die zur Herstellung der Polymerdispersionen eingesetzten Monomergemische M) können zusätzlich zur Verbindung a) wenigstens eine weitere, davon verschiedene Verbindung d) mit einer radikalisch polymerisierbaren alpha, beta-ethylenisch ungesättigten Doppelbindung und mindestens einer kationogenen und/oder kationischen Gruppe pro Molekül einpolymerisiert enthalten.

Bevorzugt handelt es sich bei den kationogenen bzw. kationischen Gruppen der Komponente d) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignete Verbindungen d) sind z. B. die von DEAEMA verschiedene Ester von alpha, beta-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente dieser Ester Acrylsäure, Methacrylsäure und deren Gemische eingesetzt.

Geeignete zusätzliche Verbindungen d) sind N,N-Dimethylaminomethyl(meth)acrylat, N,N-Dimethylaminoethyl(meth)acrylat, N,N-Diethylaminoethylacrylat, N,N-Dimethylaminopropyl(meth)acrylat, N,N-Diethylaminopropyl(meth)acrylat und N,N-Dimethylaminocyclohexyl(meth)acrylat.

Geeignete Monomere d) sind weiterhin die Amide der zuvor genannten alpha, beta-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Dazu zählen N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)-butyl]methacrylamid, N-[2-(Diethylamino)ethyl]acrylamid, N-[4-(Dimethylamino)cyclohexyl]acrylamid, N-[4-(Dimethylamino)cyclohexyl]methacrylamid etc.

Geeignete Monomere d) sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide.

Geeignete Monomere d) sind weiterhin vinyl- und allylsubstituierte Stickstoffheterocyclen, wie N-Vinylimidazol, N-Vinyl-2-methylimidazol, vinyl- und allylsubstituierte heteroaromatische Verbindungen, wie 2- und 4-Vinylpyridin, 2- und 4-Allylpyridin, und die Salze davon.

Zur Herstellung der erfindungsgemäßen wässrigen Polymerdispersionen Pd) wird das Monomer d), soweit vorhanden, vorzugsweise in einer Menge eingesetzt, dass die Summe der Mengen der Monomere a) und der Monomere d) in einem Bereich von 25 bis 65 Gew.- %, besonders bevorzugt 30 bis 60 Gew.-%, bezogen auf das Gesamtgewicht der zur Polymerisation eingesetzten Monomere, liegt.

Zur Herstellung der erfindungsgemäßen wässrigen Polymerdispersionen Pd) wird die Komponente d) vorzugsweise in einer Menge von 0 bis 50 Gew.-%, bezogen auf das Gesamtgewicht der zur Polymerisation eingesetzten Monomere, eingesetzt.

Wie bereits ausgeführt, wurde überraschenderweise gefunden, dass die erfindungsgemäßen und erfindungsgemäß eingesetzten Polymerdispersionen Pd) auf Basis von DEAEMA (Komponente a)) über ein besonders gutes Eigenschaftsprofil verfügen. Dieses Eigenschaftsprofil kann in der Regel ohne den Einsatz weiterer Monomere mit kationogenen/kationischen Gruppen erzielt werden. Eine spezielle Ausführungsform betrifft daher Polymerdispersionen Pd), die kein zusätzliches Monomer d) einpolymerisiert enthalten.

Soweit vorhanden, wird die Komponente d) vorzugsweise in einer Menge von 0,1 bis 40 Gew.-%, besonders bevorzugt 1 bis 30 Gew.-%, insbesondere 2 bis 25 Gew.-%, bezogen auf das Gesamtgewicht der zur Polymerisation eingesetzten Monomere, eingesetzt.

In einer besonders bevorzugten Ausführungsform des erfindungsgemäßen Verfahrens wird ein Monomergemisch M) eingesetzt wird, das aus
- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b), insbesondere Methylmethacrylat,
besteht.

Zur Herstellung der Polymerisate durch radikalische Emuslionspolymerisation wird hiermit ausdrücklich auf die Offenbarung der WO 2009/016258 Bezug genommen, in der die Herstellung und bevorzugte Ausführungsformen ausführlich beschrieben sind.

Die in den erfindungsgemäßen Dispersionen enthaltenen Polymere weisen vorzugsweise ein mittels Gelpermeationschromatographie bestimmtes mittleres Molekulargewicht M_{w} im Bereich von 30000 bis 500000, besonders bevorzugt 60000 bis 140000, insbesondere 80000 bis 120000 g/mol, auf.

Die in den erfindungsgemäßen Dispersionen Pd) enthaltenen Polymere weisen vorzugsweise einen K-Wert (bestimmt nach Fikentscher an einer 1%igen Lösung in N-Methylpyrrolidon (NMP)) im Bereich von 40 bis 60 auf.

Die Glasübergangstemperatur T_{G} (bestimmt mittels DSC) liegt vorzugsweise in einem Bereich von 40 bis 70 °C, besonders bevorzugt 52 bis 62 °C.

Bei den in den erfindungsgemäßen Dispersionen enthaltenen Polymeren handelt es sich im Wesentlichen um statistische Copolymere.

Der mittlere Teilchendurchmesser der in der Polymerdispersion enthaltenen Polymerteilchen (bestimmt mittels analytischer Ultrazentrifuge) liegt vorzugsweise in einem Bereich von 70 bis 200 nm, besonders bevorzugt von 80 bis 150 nm, insbesondere von 90 bis 120 nm Die Teilchengrößenverteilung ist vorzugsweise im Wesentlichen unimodal.

Der LD-Wert der erfindungsgemäßen Dispersionen, bestimmt an einer 0,01%igen Dispersion in Wasser (2,5 cm Küvette, weißes Licht) beträgt vorzugsweise wenigstens 70 %, besonders bevorzugt wenigstens 80 %. Die Bestimmung der Lichtdurchlässigkeit wird z. B. in Dieter Distler, Wässrige Polymerdispersionen, Wiley-VCH (1999), S. 40, beschrieben.

Der Feststoffgehalt der erfindungsgemäßen Dispersionen beträgt vorzugsweise 10 bis 50 Gew.-%, besonders bevorzugt 20 bis 40 Gew.-%. Im Falle einer Reinigung der Dispersion mittels Ultrafiltration weisen die erfindungsgemäßen Dispersionen vorzugsweise vor und nach der Ultrafiltration Feststoffgehalte auf, die in diesen Bereichen liegen. Selbstverständlich ist es ebenfalls möglich, eine verdünnte Polymerdispersion einer Aufkonzentrierung durch Ultrafiltration zu unterziehen.

Die erfindungsgemäß zur Geschmacksmaskierung verwendeten Dispersionen weisen z. B. auch bei einem Feststoffgehalt von 30 Gew.-% extrem niedrige Viskositäten von vorzugsweise kleiner 50 mPas, besonders bevorzugt kleiner 25 mPas und insbesondere kleiner 10 mPas auf (Werte bestimmt mittels Brookfield-Viskosimeter bei 20 °C und 100 s⁻¹). Solche niedrigen Viskositäten sind für viele Anwendungen von besonderer Bedeutung.

Die Ladung der in den erfindungsgemäßen Dispersionen enthaltenen Polymere ist abhängig vom pH-Wert der Dispersion. Der isoelektrische Punkt liegt vorzugsweise in einem pH-Bereich von etwa 7,5 bis 8,5. Die fertige Dispersion weist vorzugsweise einen pH-Wert im Bereich von 8 bis 10, besonders bevorzugt von 8,5 bis 9,5 (bei einem Feststoffgehalt von 30 Gew.-%), auf. Es ist vorteilhaft, dass der pH-Wert der fertigen Dispersion höher (stärker alkalisch) gewählt wird als ihr isoelektrischer Punkt, solange eine Auflösung oder Quellung der in der Dispersion enthaltenen Polymerteilchen nicht gewünscht ist. Bei den erfindungsgemäßen Dispersionen handelt es sich deshalb vorzugsweise um basische Dispersionen.

Die erfindungsgemäßen Polymerdispersionen zeichnen sich durch ihre pH-abhängige Löslichkeit aus. Eine Einstellung des pH-Bereichs, in dem sich die Dispersion beim Ansäuern auflöst, gelingt z. B. durch die einpolymerisierte Menge an N,N-Diethylaminoethylmethacrylat (Monomer a) sowie gegebenenfalls den Einsatz weiterer Monomere mit kationogener/kationischer Gruppen (Monomer d). Vorzugsweise lösen sich die in den erfindungsgemäßen Polymerdispersionen Pd) enthaltenen Polymere bei einem pH von höchstens 6,8, besonders bevorzugt bei einem pH von höchstens 6,0.

Gemäß einer bevorzugten Ausführungsform werden Polymerdispersionen verwendet, die ein Polymer enthalten, das
- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b)
als einzige Monomere einpolymerisiert enthält.

### Komponente B

Die erfindungsgemäßen Überzugsmittel der äusseren Schicht enthalten zusätzlich zum Polymeren ein oder mehrere Antioxidantien oder eine Kombination von Antioxidantien.

Grundsätzlich eignen sich zur Verbesserung der Freisetzungsstabilität als Antioxidantien vor allem folgende Mittel, aufgeführte Kombinationen oder weitere Kombinationen:
N-Acetylcystein, Allantoin, Arginin, Arginin + Butylhydroxytoluol, Arginin + N-Acetylcystein, Ascorbylpalmitat, Asparaginsäure, Biotin, Butylhydroxyanisol,
Butylhydroxytoluol, Butylhydroxytoluol + Calciumcarbonat, Butylhydroxytoluol + Na-EDTA,
Butylhydroxytoluol + N-Acetylcystein
   Calcium-bis[monoethyl(3,5-di-tert-butyl-4-hydroxy-benzyl)phosphonat], Catechin, Citronensäure, Cysteamin, Ethylhexylthioglykolat, Gallussäure, Hypophosphorige Säure, Kaffeesäure, Kaliumiodid, Kreatin, Kreatinin, Kupfer(I)-chlorid, Kupfer(II)-chlorid, Lysin, MEHQ, Methionin, Na-EDTA, Natriumcarbonat, Natriumdihydrogenphosphat, Dinatriumhydrogenphosphat, Natriumpropionat, Nordihydroguajaretsäure, Orotsäure, Penicillamin, Phosphorsäure, Propylgallat, Resveratrol, Riboflavin, Spermidin, Thioglykolsäure, Tocopherol, Tocopherolacetat, Trometamol, Tyrosin, Weinsäure

Grundsätzlich eigen sich zur Verbesserung der Stabilität gegen Gelbfärbung vor allem folgende Mittel, aufgeführte Kombinationen oder weitere Kombinationen:
Ölsäure, Simethicon, Butylhydroxytoluol, Natriumhydrogensulfit, Tocopherol, Natriumdihydrogencitrat, Natriumhypochlorit, Natriumhypophosphit, Dinatriumhydrogenphosphat, Tocopherol, Tocopherolacetat, Arginin,
Butylhydroxytoluol + Na-EDTA, Acetylcystein (N-Acetylcystein), Butylhydroxytoluol,
Allantoin, Butylhydroxyanisol, Natriumcarbonat, Cysteamin, N-Acetylcystein

Bevorzugte Antioxidantien sind Verbindungen vom Phenoltyp. Bevorzugte phenolische Verbindungen sind beispielsweise Butylhydroxytoluol oder Butylhydroxyanisol, da sie sowohl die Auflösungsverzögerung als auch die Gelbfärbung völlig verhindern. Weitere geeignete Produkte sind: Catechin, Gallussäure oder deren Ester, Tocopherol, Kaffeesäure, Hydrochinonmonomethylether (MEHQ), Nordihydroguajaretsäure, Resveratrol.

Ebenso bevorzugte Antioxidantien sind thiolische Verbindungen , wie N-Acetylcystein Cysteamin, Thioglykolsäure.

Weiterhin bevorzugt sind basische Aminosäuren wie Arginin und Lysin.

Bevorzugte Antioxidantien sind auch Alkalicarbonate oder Alkalibicarbonate, insbesondere die Natriumssalze, bevorzugt Natriumcarbonat.

Bevorzugt sind auch Kombinationen mit EDTA, insbesondere Na-EDTA oder mit Citronensäure.

Besonders bevorzugt sind N-Acetylcystein, Arginin, Lysin, Butylhydroxytoluol, Butylhydroxytoluol + Na EDTA, sowie Natriumcarbonat oder Kombinationen davon.

Alle genannten Verbindungen oder Klassen von Verbindungen können auch in Kombination verwendet werden.

Die Antioxidantien werden in Mengen von 0.1 bis 30, bevorzugt 0.3 bis 20, besonders bevorzugt 0.5 bis 12 Gew.-%, bezogen auf die Gesamtmenge des Feststoffes des Überzugsmittels eingesetzt.

### Komponente C

Weiterhin enthalten die erfindungsgemäßen Überzugsmittel als Komponente C Weichmacher, bevorzugt lipophile Weichmacher. Besonders geeignete Weichmacher sind Tributylcitrat, Acetyltributylcitrat, Acetyltriethylcitrat, Triacetin, Triethylcitrat, Diethylsebacat und Dibutylsebacat.

### Komponenten D

Die erfindungsgemäß verwendeten Überzugsmittel für pharmazeutische Darreichungsformen können als Komponenten D zusätzlich wenigstens einen weiteren pharmazeutisch akzeptablen Hilfsstoff enthalten. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. Ph. Eur., USP, JP) gelisteten sowie andere Hilfsstoffe, deren Eigenschaften einer physiologischen Anwendung nicht entgegenstehen.

Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Porenbildner, Neutralisierungsmittel, Glanzmittel, Farbstoffe, Pigmente, Desinfektions- oder Konservierungsmittel, Verdickungsmittel, etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Übliche Mengen der Hilfsstoffe liegen in einem Bereich von jeweils 0 bis 70 Gew.-%, bevorzugt 0 bis 60 Gew.-%, insbesondere 1 bis 50 Gew.-%, bezogen auf das Gesamtgewicht des Feststoffes des Überzugsmittels.

Die Überzugsmittel, die zur Herstellung der der äusseren Überzugsschicht eingesetzt werden, können, bezogen auf das Gesamtgewicht der wässrigen Dispersion
i) 1 bis 45 Gew.-% Komponente A,
ii) 0,01 bis 15 Gew.-% Komponente B,
iii) 0,1 bis 15 Gew.-% Komponente C,
iv) 0 bis 35 Gew.-% Komponenten D, enthalten.

Bevorzugte Überzugsmittel enthalten
i) 1,5 bis 42,5 Gew.-% Komponente A,
ii) 0,02 bis 10 Gew.-% Komponente B,
iii) 0,15 bis 12,5 Gew.-% Komponente C,
iv) 0 bis 30 Gew.-% Komponenten D.

Besonders bevorzugte Überzugsmittel enthalten, bezogen auf das Gesamtgewicht der Dispersion
i) 4 bis 40 Gew.-% Komponente A,
ii) 0.05 bis 6 Gew.-% Komponente B,
iii) 0,4 bis 8 Gew.-% Komponente C,
iv) 0.1 bis 20 Gew.-% Komponenten D.

Die Herstellung der Überzugsmittel sowohl der inneren wie der äusseren Schicht kann z. B. durch inniges Vermischen einer erfindungsgemäß verwendeten Polymerdispersion oder Polymerlösung oder eines daraus durch ein Trocknungsverfahren erhältlichen Polymers erfolgen.

Die erfindungsgemäßen Überzugsmittel können z. B. in Pulverform, als Schmelze oder in wässriger Emulsion durch Granulieren, Gießen, Ausstreichen oder mittels Sprühauftrag angewendet werden. Bevorzugt ist die Anwendung als Polymerdispersion, speziell als Primärdispersion. Die erfindungsgemäßen Überzugsmittel können zusätzlich wenigstens eine weitere Polymerkomponente enthalten. Dabei können Mischungen aus wenigstens zwei Dispersionen, wenigstens einer Dispersion und wenigstens einer Lösung, wenigstens einer Dispersion und wenigstens einem Pulver, wenigstens zwei Pulvern, etc. zum Einsatz kommen.

Die erfindungsgemäßen Überzugsmittel eignen sich für Dosierungsformen grundsätzlich beliebiger pharmazeutischer Wirkstoffe mit sauren Gruppen, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen- Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

### Beispiele geeigneter saurer Wirkstoffe sind:

Aceclofenac, Acediasulfon, Acedoben, Aceglutamid, Acemetacin, Acetoxyvaleriansäure, Acetylcystein, Acetylleucin, Acetylmethionin, Acetylsalicylsäure, Acexamsäure, Acifluorfen, Acipimox, Acitazanolast, Acitretin, Aconiazid, Acrivastin, Actarit, Adapalen, Adipiodon, Alacepril, Alatrofloxacin, Alclofenac, Alitam, Alitretinoin, Alliin, Alminoprofen, Alprostadil, Alvimopan, Amfenac, Amineptin, Aminocapronsäure, Aminohippursäure, 5-Aminolevulinsäure, Aminomethylbenzoesäure, Aminopyralid, Aminosalicylsäure, Amlexanox, Amoxicillin, Ampicillin, Amphotericin B, Argatroban, Arsenamid, Artesunat, Aspartame, Aspoxicillin, Atorvastatin, Aurintricarboxysäure, Aurothioäpfelsäure, Aviglycin, Aviptadil, Azelainsäure, Azidocillin, Azlocillin, Azoximer bromide, Aztreonam, Baclofen, Balofloxacin, Balsalazide, Benazepril, Benazolin, Bendamustine, Bendazac, Bensuldazinsäure, Bensulfuron, Bentazon, Bentiacid, Bentiromid, Benzylpenicillin, Phenoxymethylpenicillin, Bepotastin, Beraprost, Betamipron, Betanin, Bexarotene, Bezafibrat, Bilanafos, Biotin, Bivalirudin, beta-Boswellinsäure, Bromfenac, Bucillamin, Bumadizon, Bumetanid, Buthiopurin, Butibufen, C12-Peptide, Calcitonin, Canrenonsäure, Capobeninsäure, Captopril, Carbenicillin, Carbenoxolon, Carbidopa, Carbocistein, Carboprost, Carfecillin, Carfentrazon, Cargluminsäure, Carindacillin, Carnosin, Carprofen, Carumonam, Carzenid, Cefacetril, Cefaclor, Cefadroxil, Cefalexin, Cefaloglycin, Cefaloridine, Cefalotin, Cefamandol, Cefapirin, Cefatrizin, Cefazolin, Cefbuperazon, Cefdinir, Cefepim, Cefixim, Cefmenoxim, Cefmetazol, Cefminox, Cefodizim, Cefonicid, Cefoperazon, Ceforanid, Cefoselis, Cefotaxim, Cefotetan, Cefotiam, Cefovecin, Cefoxazol, Cefoxitin, Cefozopran, Cefpimizol, Cefpiramid, Cefprozil, Cefradin, Cefroxadin, Cefsulodin, Ceftazidim, Ceftezol, Ceftibuten, Ceftiofur, Ceftizoxim, Ceftriaxon, Cefuroxim, Cefuzonam, Ceruletid, Cetirizin, Cetraxat, Chenodeoxycholsäure, Chloramben, Chlorambucil, Chondroitinsulfat, Chromocarb, Ciclacilin, Cicloxilinsäure, Cilastatin, Cilazapril, Cinametinsäure, Cinoxacin, Ciprodex, Ciprofibrat, Ciprofloxacin, Clanobutin, Clavulansäure, Clidanac, Clinofibrat, Clofencet, Clonixin, Cloprop, Clopyralid, Clorazepat, Cloxacillin, Cloxyfonac, Corticorelin, Cromoglicinsäure, Cyclanilid, Cyclobutyrol, Cynarin, Dalapon, Daminozid, Danofloxacin, Daptomycin, Deferasirox, Dehydrocholsäure, Delapril, Dexibuprofen, Dexketopropfen, Dexlipotam, Dextrothyroxin, Diacerein, Dibromotyrosin, Dicamba, Dichlorprop, Dichlorprop-P, Diclofenac, Diclofop, Dicloxacillin, Difenoxin, Difloxacin, Diflufenzopyr, Diflunisal, Diiodotyrosine, Dikegulac, Dimecrotinsäure, Dinoprost, Dinoproston, Divalproex, D-Luciferin, Doconexent, Doripenem, D-Penicillamin, Droxidopa, Ecabet, Eflornithin, Eltenac, Eltrombopag, Enalapril, Enalaprilat, Endothal, Enfenaminsäure, Enfuvirtid, Enkorten, Enoxacin, Enoxolon, Enrofloxacin, Epalrestat, Epoetin alfa, Epoprostenol, Eprosartan, Eptifibatid, Erdostein, Ertapenem, Erythrosine, Escin, Etacrynsäure, Etodolac, Exenatid, Faropenem, Febuxostat, Felbinac, Fenbufen, Fenchlorazol, Fencibutirol, Fendizoinsäure, Fenofibrinsäure, Fenoprofen, Fenoxaprop, Fenoxaprop-P, Fentiazac, Fexofenadin, Flavodinsäure, Fleroxacin, Flomoxef, Fluazifop, Fluazifop-P, Flucloxacillin, Flufenaminsäure, Flumequin, Flumiclorac, Flunixin, Flunoxaprofen, Fluorodopa F18, Flupropanat, Fluprostenol, Flupyrsulfuron, Flurbiprofen, Folic säure, Folinsäure, Fostosal, Fosinopril, Fudostein, Fumagillin, Furosemid, Fusidinäure, Gabapentin, Gadobeninsäure, Gadobutrol, Gadodiamid, Gadofosveset, Gadopentetinsäure, Gadoterinsäure, Gadoxetinsäure, Gamma-aminobuttersäure, Gamolensäure, Garenoxacin, Gatifloxacin, Gemfibrozil, Gemifloxacin, Gentisinsäure, Gibberrellinsäure, Gibberellin A4, Gibberellin A7, Glucagon, Glufosinat, Glycopin, Glycyrrhizin, Glyphosat, Halazon, Hetacillin, Hyaluronsäure, Ibafloxacin, Ibuprofen, Icatibant, Icosapent, Iloprost, Imazamox, Imazapic, Imazapyr, Imazaquin, Imazethapyr, Imidapril, Imipenem, Indium In-111 pentetreol, Indobufen, Indol-3-ylessigsäure, Indolebuttersäure, Indometacin, Invicorp, locetamsäure, lodamidelodoxamsäure, loglicinsäure, lopansäure, lotalamsäure, lotroxinsäure, loxaglic säure, loxitalamic säure, Ipodinsäure, Isotretinoin, karetazan, alpha-Ketoglutarsäure, Ketoprofen, Ketorolac, Lactobionsäure, Lasalocid A, Latamoxef, Letostein, Levo carnitinacetate, Levocabastin, Levocetirizin, Levofloxacin, Levofolinsäure, Levomefolsäure, Levopropicillin, Levothyroxin, Limaprost, Liothyronin, Liraglutid, Lisinopril, Lobenzarit, Lodoxamid, Lomefloxacin, Lonazolac, Lonidamin, Loracarbef, Loxoprofen, Lubiproston, Lumiracoxib, Luprostiol, Lusupultid, Lymecyclin, Marbofloxacin, Mecillinam, Meclofenaminsäure, Mecoprop-P, Mefenaminsäure, Mefenpyrdiethyl, Mefolinsäure, Meglutol, Meloxicam, Melphalan, Mepifyllin, Meropenem, Mesalazin, Metadoxin, Metampicillin, Methotrexat, Methyldopa, Metiazinic säure, Meticillin, Metirosin, Metsulfuron, Mezlocillin, Midoriamin, Miloxacin, Mitiglinid, Moexipril, Mofezolac, Monensin, Montelukast, Moxifloxacin, Mupirocin, Mycophenolsäure, Nadifloxacin, Nadroparin, Nafcillin, Nalidixinsäure, Naphthalen-1-essigsäure, Naproxen, Naptalam, Narasin, Natamycin, Nateglinid, Nedocromil, Nesiritid, Nicotinsäure, Nifluminsäure, Nisin, Norfloxacin, N-phenylphthalaminsäure, Nystatin, Ofloxacin, Olopatadine, Olsalazin, Orazamid, Orbifloxacin, Orotsäure, Oxaceprol, Oxacillin, Oxaprozin, Oxitriptan, Oxolinsäure, Ozagrel, Pamoin säure, Panipenem, Pantothensäure, Parahydroxyzimtsäure, Pasiniazid, Pazufloxacin, Pefloxacin, Peforelin, Pelargonsäure, Pemetrexed, Penicillin G, Peramivir, Perindopril, Phenethicillin, Phenoxymethylpenicillin, Photofrin, Phthalylsulfathiazol, Picloram, Pidolinsäure, Pidotimod, Pipemidinsäure, Piperacillin, Pirenoxin, Piretanid, Piromidinsäure, Piroxicam, Pirprofen, Pivagabin, Pralatrexat, Pranoprofen, Pregabalin, Primisulfuron-Methyl, Probenecid, Procodazol, Proglumid, Prohexadion, Propagermanium, Propicillin, Protizinsäure, Prulifloxacin, Pyrazosulfuron, Pyrithiobac, Quinapril, Quinaprilat, Quinclorac, Quinmerac, Quizalofop, Quizalofop-P, Raltitrexed, Ramatroban, Ramipril, Ranelinsäure, Rebamipide, Repaglinide, Rhodamine B, Ritiometan, Robenacoxib, Romurtid, Rosoxacin, Rotraxat, (R)-Trolox, Rufloxacin, Salicylsäure, Salinomycin, Salsalat, Sarafloxacin, Sarpogrelat, Semax, Semduramicin, Seratrodast, Sincalid, Sivelestat, Sofalcon, Somatorelin, Somatostatin, Sorbinsäure, Spagluminsäure, Sparfloxacin, Spirapril, Stepronin, Stibogluconsäure, (S)-Trolox, Sugammadex, Sulbactam, Sulbenicillin, Sulfasalazin, Sulfasuccinamid, Sulfometuron, Sulfosat, Sulindac, Suprofen, Suxibuzon, Talaporfin, Tamibaroten, Tazobactam, Tecloftalam, Teicoplanin, Telmestein, Telmisartan, Temocapril, Temocillin, Tenoinsäure, Teriparatid, Tetracosactid, Thioctsäure, Thiomersal, Thymalfasin, Tiagabin, Tianeptin, Tiaprofensäure, Tiaprost, Ticarcillin, Tidiacin, Timonacin, Tiopronin, Tiratricol, Tirofiban, Tocamphyl, Tolfenaminsäure, Tolmetin, Tolylphthalaminsäure, Tosufloxacin, Trafermin, Trandolapril, Tranexamsäure, Tranilast, Trepibuton, Treprostinil, Tretinoin, Tribenuron, Triclopyr, Tridecactid, Triflusal, Triflusulfuron, Trolox, Tropesin, Trovafloxacin, Ubenimex, Undecylensäure, Ursodeoxycholsäure, Valeriansäure, Valproinsäure, Valsartan, Vancomycin, Vebufloxacin, Vedaprofen, Verteporfin, Vigabatrin, Zaltoprofen, Zanamivir, Ziconotid, Zofenopril

Viele dieser Wirkstoffe weisen einen bitteren oder unangenehmen Geschmack auf.

Die Wirkstoffe können gewünschtenfalls auch in Form ihrer pharmazeutisch akzeptablen Modifikationen oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemäßen Zusammensetzungen auch zwei oder mehrere pharmazeutische Wirkstoffe enthalten.

Erfindungsgemäß können die Überzugsmittel zur Beschichtung von saure Wirkstoffe enthaltenden Kernen in Form von Extrudaten, Minitabletten, Granulaten, Pellets, Mikropellets oder Kristallen eingesetzt werden. Die Kerne können auch aus Nonpareilles bestehen, die mit dem sauren Wirkstoff beschichtet sind.

Zur Herstellung von Dosierungsformen können die überzogenen wirkstoffhaltigen Kerne mit geeigneten Hilfsstoffen abgemischt und zu Tabletten verpresst werden, die im wässrigen Milieu der Mundhöhle zerfallen und die gecoateten feinen Formlinge wieder freigeben. Besondere Bedeutung haben hierbei so genannte Oral Dispersibles, d. h. Tabletten, die im Mund innerhalb von kurzer Zeit zerfallen und die mit den erfindungsgemäßen Überzügen versehenen kleinen Formlinge freisetzen.

Weiterhin können die Überzugsmittel auch vorteilhaft zum Überziehen von Tabletten eingesetzt werden.

Die hervorragende Geschmacksmaskierung resultiert aus der Unlöslichkeit der erfindungsgemäßen Polymeren bei pH-Werten größer 6 und der schnellen Löslichkeit bei pH-Werten unter 6. Das heißt im Speichel (pH-Wert: 7,2) sind entsprechend überzogene Formen sehr lange stabil und es erfolgt kein Kontakt des bitteren Arzneistoffs mit der Mundschleimhaut, aber im Magen bei pH-Werten von 1 bis 5 erfolgt eine sehr schnelle Freisetzung des Wirkstoffs. Die Auflösung ist dabei so schnell, dass kein Unterschied im Wirkungseintritt vorhanden ist, verglichen mit einer nicht gecoateten Form. In der Regel lösen sich Filmüberzüge eines erfindungsgemäßen Polymers innerhalb von 5 min in Magensaft auf, wohingegen sie in Phosphatpuffer pH 7,2 über 2 Stunden stabil sind. Überraschenderweise lösen sich die Filmüberzüge auch in Medien mit pH-Werten von 4,5 relativ schnell, so dass die daraus hergestellten Darreichungsformen auch bei anaciden Patienten bzw. Patienten, die mit Antacida behandelt werden, eine schnelle Wirkung entfalten.

Die erfindungsgemäßen Überzugsmittel weisen eine gute Stabilität gegen Veränderung der Freisetzung unter Temperaturbelastung auf. In vielen Fällen ist auch die Stabilität gegen Gelbfärbung ausgeprägt. Weiterhin wird auch die Wasserdampfpermeabilität vorteilhaft beeinflusst.

### Beispiele

Verwendete Abkürzungen:
BHT: Butylhydroxybenzol
NAC: N-Acetylcystein
ATBC: Acetyltributylcitrat
TEC: Triethylcitrat
MEHQ: Hydrochinomonomethylether
d: Tage
VE: vollentsalzt

Ludipress®: formuliertes Produkt aus Lactose (90%), Povidon (3,5%) und Crospovidon (3,5%) Avicel® PH 102: mikrokristalline Cellulose
Kollidon® VA 64: Vinylpyrrolidon - Vinylacetat (6:4) Copolymer (Copovidon)
Kollidon® CL: quervernetztes Polyvinylpyrrolidon (Crospovidon)
Kollicoat® IR: Polyvinylalkohol- Polyethylenglykol 6000-Pfropfpolymer (Gewichtsverhältnis PVA:PEG 75:25, Verseifungsgrad 94 mol-%), Molgewicht: 45000 Dalton

Alle Angaben in % beziehen sich, wenn nicht anders angegeben, auf Gew.-%.

Kationische Polymere:
Die Herstellung der Polymere erfolgt analog Beispiel 1 der WO 2009/016258.
Polymer A: Methylmethacrylat/Diethylaminoethylmethacrylat, Gew.v. 60:40,
Polymer B: Methylmethacrylat/Diethylaminoethylmethacrylat, Gew.v. 55:45
Polymer C: Methylmethacrylat/Diethylaminoethylmethacrylat, Gew.v. 53:47
Die K-Werte gemessen 0.1 gew.-ig in NMP betrugen 50+/- 0.5
Die Polymere wurden als 30 gew.-%ige wässrige Dispersionen mit einem pH-Wert von 9+/- 0.3 eingesetzt. Die mittlere Partikelgröße der Primärdispersion betrug 110 nm.

Die Bestimmung der Freisetzung aus Darreichungsformen erfolgte mit dem in der Pharmakopoe der USA (USP 32) unter Dissolution beschriebenen Gerät mit Paddlerührer.

### Beispiel 1

### Gecoatete Acetylsalicylsäuretablette 100mg

**Rezeptur der Tablette:**

| | |
|---|---|
| Ludipress | 189mg |
| Avicel PH 101 | 40mg |
| Acetylsalicylsäure | 100mg |
| Stearinsäure | 1mg |
| Tablettengewicht | 330mg |
| | |
| Verfahren | Direkttablettierung |
| Tablettenform | gewölbt |
| Durchmesser | 9mm |

**Sprührezeptur innere Schicht:**

| | |
|---|---|
| Kollicoat IR | 12% |
| Talkum | 8% |
| VE-Wasser | 80% |
| Feststoffgehalt | 20% |

**Coatingbedingungen:**

| Maschine | Accela Cota 24 / Horizontaltrommelcoater |
|---|---|
| Zulufttemperatur | 55°C |
| Sprühdruck | 2,5 bar |
| Formierluftdruck | 1 bar |
| Sprühdüse | Schlick 930 / 1mm |
| Zuluftmenge | 200 m³/h |
| Beladung | 7 kg |
| Sprührate | 30 g/min. |
| Auftragsmenge | 1,5 mg/cm² |

**Sprührezeptur äußere Schicht:**

| | |
|---|---|
| Polymer B (30%ige Dispersion) | 33,33% |
| Tributylcitrat | 1,50% |
| Butylhydroxytoluol | 0,5 |
| Talkum | 6% |
| Titandioxid | 2% |
| VE-Wasser | 56,67% |
| Feststoffgehalt | 20% |

**Coatingbedingungen:**

| Maschine | Accela Cota 24 / Horizontaltrommelcoater |
|---|---|
| Zulufttemperatur | 55°C |
| Sprühdruck | 2 bar |
| Formierluftdruck | 1 bar |
| Sprühdüse | Schlick 930 / 1 mm |
| Zuluftmenge | 200 m³/h |
| Beladung | 7 kg |
| Sprührate | 30 g/min. |
| Auftragsmenge | 4,5 mg/cm² |

### Ergebnis:

**Freisetzung in Acetatpuffer pH 4,5**

| Zeit in min. | Ausgangswert in % | 3 Monate 30/70% r.F. in % | 3 Monate 40°C in % |
|---|---|---|---|
| 15 | 73,5 | 74,6 | 73,9 |
| 30 | 99,8 | 98,9 | 99,5 |
| 60 | 100,2 | 100,3 | 99,7 |

**Freisetzung in Phosphatpuffer pH 6,8**

| Zeit in min. | Ausgangswert in % | 3 Monate 30/70% r.F. in % | 3 Monate 40°C in % |
|---|---|---|---|
| 15 | 0,2 | 0,3 | 0,4 |
| 30 | 0,3 | 0,3 | 0,3 |
| 60 | 0,4 | 0,5 | 0,6 |

**Weitere Eigenschaften**

| | Ausgangswert | 3 Monate 30/70% r.F. | 3 Monate 40°C |
|---|---|---|---|
| Aussehen | Glatt, glänzend, kein Kleben, keine Beschädigungen | Glatt, glänzend, kein Kleben, keine Beschädigungen | Glatt, glänzend, kein Kleben, keine Beschädigungen |
| Geschmackstest | Kein bitterer Geschmack nach 5 min im Mund | Kein bitterer Geschmack nach 5 min im Mund | Kein bitterer Geschmack nach 5 min im Mund |

### Vergleichsbeispiel zu 1

Herstellung analog Beispiel 1 aber ohne die innere Schicht

**Freisetzung in Acetatpuffer pH 4,5**

| Zeit in min. | Ausgangswert in % | 3 Monate 30/70% r.F. in % | 3 Monate 40°C in % |
|---|---|---|---|
| 15 | 72,5 | 81,5 | 89,7 |
| 30 | 99,6 | 98,5 | 99,1 |
| 60 | 99,8 | 100,3 | 99,9 |

**Freisetzung in Phosphatpuffer pH 6,8**

| Zeit in min. | Ausgangswert in % | 3 Monate 30/70% r.F. in % | 3 Monate 40°C in % |
|---|---|---|---|
| 15 | 0,4 | 12,3 | 10,6 |
| 30 | 1,8 | 25,7 | 23,4 |
| 60 | 3,9 | 54,8 | 49,8 |

**Weitere Eigenschaften**

| | Ausgangswert | 3 Monate 30/70% r.F. | 3 Monate 40°C |
|---|---|---|---|
| Aussehen | Glatt, glänzend, kein Kleben, keine Beschädigungen | Rau, matt, Tabletten kleben, deutliche Beschädigungen | Rau, matt, Tabletten kleben, deutliche Beschädigungen |
| Geschmackstest | Kein bitterer Geschmack nach 5 min im Mund | Bitterer Geschmack nach 0,5 min im Mund | Bitterer Geschmack nach 0,5 min im Mund |

### Beispiel 2

Gecoatete Ibuprofentablette 200mg

**Rezeptur der Tablette:**

| Ibuprofen | 200mg |
|---|---|
| Avicel PH 101 | 142,5mg |
| Kollidon CL | 10 |
| Aerosil 200 | 6 |
| Magnesiumstearat | 1,5mg |
| Tablettengewicht | 360mg |
| | |
| Verfahren | Direkttablettierung |
| Tablettenform | gewölbt |
| Durchmesser | 9mm |

**Sprührezeptur innere Schicht:**

| | |
|---|---|
| Kollicoat IR | 12% |
| Talkum | 8% |
| VE-Wasser | 80% |
| Feststoffgehalt | 20% |

**Coatingbedingungen:**

| Maschine | Accela Cota 24 / Horizontaltrommelcoater |
|---|---|
| Zulufttemperatur | 55°C |
| Sprühdruck | 2,5 bar |
| Formierluftdruck | 1 bar |
| Sprühdüse | Schlick 930 / 1 mm |
| Zuluftmenge | 200 m³/h |
| Beladung | 7 kg |
| Sprührate | 30 g/min. |
| Auftragsmenge | 1,5 mg/cm² |

**Sprührezeptur äußere Schicht:**

| | |
|---|---|
| Polymer A (30%ige Dispersion) | 33,33% |
| Acetyltriethylcitrat | 1,50% |
| Butylhydroxytoluol | 0,5 |
| Talkum | 6% |
| Titandioxid | 2% |
| VE-Wasser | 56,67% |
| Feststoffgehalt | 20% |

**Coatingbedingungen:**

| Maschine | Accela Cota 24 / Horizontaltrommelcoater |
|---|---|
| Zulufttemperatur | 55°C |
| Sprühdruck | 2 bar |
| Formierluftdruck | 1 bar |
| Sprühdüse | Schlick 930 / 1 mm |
| Zuluftmenge | 200 m³/h |
| Beladung | 7 kg |
| Sprührate | 30 g/min. |
| Auftragsmenge | 4,5 mg/cm² |

### Ergebnis:

**Freisetzung in Acetatpuffer pH 4,5**

| Zeit in min. | Ausgangswert in % | 3 Monate 30/70% r.F. in % | 3 Monate 40°C in % |
|---|---|---|---|
| 15 | 43,4 | 44,6 | 42,9 |
| 30 | 81,8 | 82,6 | 80,5 |
| 60 | 98,9 | 99,9 | 99.2 |

**Freisetzung in Phosphatpuffer pH 6,8**

| Zeit in min. | Ausgangswert in % | 3 Monate 30/70% r.F. in % | 3 Monate 40°C in % |
|---|---|---|---|
| 15 | 0,1 | 0,2 | 0,4 |
| 30 | 0,3 | 0,5 | 0,5 |
| 60 | 0,5 | 0,7 | 0,5 |

**Weitere Eigenschaften**

| | Ausgangswert | 3 Monate 30/70% r.F. | 3 Monate 40°C |
|---|---|---|---|
| Aussehen | Glatt, glänzend, kein Kleben, keine Beschädigungen | Glatt, glänzend, kein Kleben, keine Beschädigungen | Glatt, glänzend, kein Kleben, keine Beschädigungen |
| Geschmackstest | Kein bitterer Geschmack nach 5 min im Mund | Kein bitterer Geschmack nach 5 min im Mund | Kein bitterer Geschmack nach 5 min im Mund |

### Beispiel 3:

Gecoatete Ibuprofen-Minipellets

**Zusammensetzung der Pellets**

| Substanz | Zusammensetzung pro Pellet [%] |
|---|---|
| Ibuprofen | 100 |

Pelletgröße 200 bis 400 µm

### Zusammensetzung der Sprührezeptur

**Sprührezeptur innere Schicht:**

| | |
|---|---|
| Kollicoat IR | 12% |
| Talkum | 8% |
| VE-Wasser | 80% |
| Feststoffgehalt | 20% |

### Coatingparameter

Gecoatet wurde in einem Wirbelschichtgranulator "Glatt GPCG 3.1" der Fa. Glatt.

| | |
|---|---|
| Sprühdüse | 1 mm Durchmesser |
| Anzahl der Sprühdüsen | 1 |
| Befüllung | 2,5 kg Ibuprofenpellets 200-400µm |
| Verfahren | Bottom-Spray (Wurster) |
| Sprühdruck | 2,0 bar |
| Zulufttemperatur | 55 °C |
| Ablufttemperatur | 39°C |
| Sprührate | 15 g/min. |
| Trocknung | ca. 5 min. |
| Auftragsmenge/Gewichtszuwachs | 5 % |

**Sprührezeptur äußere Schicht:**

| | |
|---|---|
| Polymer B (30%ige Dispersion) | 33,33% |
| Triethylcitrat | 1,50% |
| Gelbes Eisenoxid | 0,5 |
| Talkum | 6% |
| Titandioxid | 2% |
| VE-Wasser | 56,67% |
| Feststoffgehalt | 20% |

### Coatingparameter

Gecoatet wurde in einem Wirbelschichtgranulator "Glatt GPCG 3.1" der Fa. Glatt.

| | |
|---|---|
| Sprühdüse | 1 mm Durchmesser |
| Anzahl der Sprühdüsen | 1 |
| Befüllung | 2,5 kg Ibuprofenpellets 200-400µm |
| Verfahren | Bottom-Spray (Wurster) |
| Sprühdruck | 1,0 bar |
| Zulufttemperatur | 45 °C |
| Ablufttemperatur | 29°C |
| Sprührate | 15 g/min. |
| Trocknung | ca. 5 min. |
| Auftragsmenge/Gewichtszuwachs | 20 % |

Nach dem Coating wurde die Pellets mit 0,2 % Aerosil 200 für 10 min in einem Turbulamischer gemischt.

### Ergebnis:

**Freisetzung in Acetatpuffer pH 4,5**

| Zeit in min. | Ausgangswert in % | 3 Monate 30/70% r.F. in % | 3 Monate 40°C in % |
|---|---|---|---|
| 15 | 58,2 | 60,0 | 58,1 |
| 30 | 89,5 | 91,2 | 90,5 |
| 60 | 99,7 | 99,9 | 99.2 |

**Freisetzung in Phosphatpuffer pH 6,8**

| Zeit in min. | Ausgangswert in % | 3 Monate 30/70% r.F. in % | 3 Monate 40°C in % |
|---|---|---|---|
| 15 | 0,8 | 0,9 | 1,1 |
| 30 | 1,5 | 1,9 | 1,6 |
| 60 | 2,6 | 2,8 | 2,9 |

**Weitere Eigenschaften**

| | Ausgangswert | 3 Monate 30/70% r.F. | 3 Monate 40°C |
|---|---|---|---|
| Aussehen | Glatt, kein Kleben, keine Beschädigungen | Glatt, kein Kleben, keine Beschädigungen | Glatt, kein Kleben, keine Beschädigungen |
| Geschmackstest | Kein bitterer Geschmack nach 5 min im Mund | Kein bitterer Geschmack nach 5 min im Mund | Kein bitterer Geschmack nach 5 min im Mund |

### Beispiel 4

Gecoatete Ketoprofenpellets

**Rezeptur der Pellets:**

| | |
|---|---|
| Ketoprofen | 20% |
| Avicel PH 101 | 40% |
| Granulac 230 | 40% |
| | |
| Verfahren | Extrudierung, Sphäronisierung |
| Pellet-Durchmesser | 0,7-1,4 mm |

Zusammensetzung der Sprührezeptur

**Sprührezeptur innere Schicht:**

| | |
|---|---|
| Kollicoat Protect | 12% |
| Talkum | 8% |
| VE-Wasser | 80% |
| Feststoffgehalt | 20% |

**Coatingbedingungen für die innere Schicht:**

| Maschine | Aeromatic Strea / Wurstereinsatz |
|---|---|
| Zulufttemperatur | 60°C |
| Sprühdruck | 2,0 bar |
| Düsendurchmesser | 0,8 mm |
| Zuluftmenge | 85-100 m³ |
| Beladung | 0,5 kg |
| Sprührate | 8 g/min |
| Auftragsmenge | 1,0 mg/cm² |

**Sprührezeptur äußere Schicht:**

| | |
|---|---|
| Polymer C (30%ige Dispersion) | 33,33% |
| Tributylcitrat | 1,50% |
| Sunset Yellow Al-lack | 0,5 |
| Talkum | 6% |
| Titandioxid | 2% |
| VE-Wasser | 56,67% |
| Feststoffgehalt | 20% |

**Coatingbedingungen für die äußere Schicht:**

| Maschine | Aeromatic Strea / Wurstereinsatz |
|---|---|
| Zulufttemperatur | 52°C |
| Sprühdruck | 1,3 bar |
| Düsendurchmesser | 0,8 mm |
| Zuluftmenge | 85-100 m³ |
| Beladung | 0,5 kg |
| Sprührate | 8 g/min |
| Auftragsmenge | 4,5 mg/cm² |

Nach dem Coating wurde die Pellets mit 0,2% Aerosil 200 für 10 min in einem Turbulamischer gemischt.

### Ergebnis:

**Freisetzung in Acetatpuffer pH 4,5**

| Zeit in min. | Ausgangswert in % | 3 Monate 30/70% r.F. in % | 3 Monate 40°C in % |
|---|---|---|---|
| 15 | 41,2 | 39,9 | 40,5 |
| 30 | 75,4 | 74,5 | 76,6 |
| 60 | 98,6 | 97,7 | 99.5 |

**Freisetzung in Phosphatpuffer pH 6,8**

| Zeit in min. | Ausgangswert in % | 3 Monate 30/70% r.F. in % | 3 Monate 40°C in % |
|---|---|---|---|
| 15 | 0,8 | 0,9 | 0,6 |
| 30 | 1,3 | 1,2 | 1.1 |
| 60 | 1,8 | 2,0 | 1,5 |

**Weitere Eigenschaften**

| | Ausgangswert | 3 Monate 30/70% r.F. | 3 Monate 40°C |
|---|---|---|---|
| Aussehen | Glatt, kein Kleben, keine Beschädigungen | Glatt, kein Kleben, keine Beschädigungen | Glatt, kein Kleben, keine Beschädigungen |
| Geschmackstest | Kein bitterer Geschmack nach 5 min im Mund | Kein bitterer Geschmack nach 5 min im Mund | Kein bitterer Geschmack nach 5 min im Mund |

## Patentansprüche

1. Mit protektiven Überzügen versehenen Dosierungsformen, in denen ein mindestens einen sauren Wirkstoff enthaltender Kern mit mindestens einer inneren und einer äußeren Überzugsschicht versehen ist, wobei die äussere Schicht als Komponente A ein kationisches Polymer, welches ein Emulsionspolymerisat aus N,N-Diethylaminoethylmethacrylat und weiteren Monomeren darstellt, enthält, und die innere Überzugsschicht aus einem neutralen wasserlöslichen Polymer und optional Pigmenten und weiteren pharmazeutisch üblichen Hilfsstoffen besteht und als neutrale Polymere der inneren Überzugsschicht Polyvinylalkohole, Polyalkylenglykol - Polyvinylalkohol-Pfropfcopolymere, Polyvinylpyrrolidone, Vinylpyrrolidon - Vinylacetat-Copolymere, alkylierte und hydroxyalkylierte Cellulosen oder Stärken oder Gemische solcher Polymere eingesetzt werden.

2. Dosierungsformen nach Anspruch 1, enthaltend als saure Wirkstoffe, Wirkstoffe mit freien Carboxyl-, Sulfonsäure oder Phosphonsäuregruppen, aciden Hydroxylgruppen, aciden N-H Gruppen oder aciden C-H Gruppen oder Gemische solcher Wirkstoffe.

3. Dosierungsformen nach Anspruch 1 oder 2, enthaltend saure Wirkstoffe mit einem pks-Wert zwischen 6,5 und 0,5.

4. Dosierungsformen nach einem der Ansprüche 1 bis 3, enthaltend saure Wirkstoffe mit einem pKs-Wert zwischen 5,0 und 1,0.

5. Dosierungsformen nach einem der Ansprüche 1 bis 4, enthaltend als neutrale Polymere der inneren Überzugsschicht Polyvinylalkohole, Polyalkylenglykol - Polyvinylalkohol-Pfropfcopolymere oder Mischungen davon.

6. Dosierungsformen nach einem der Ansprüche 1 bis 5, enthaltend als Komponente A der äusseren Überzugsschicht ein durch radikalische Polymerisation erhaltenes Polymer aus
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,

7. Dosierungsformen nach einem der Ansprüche 1 bis 6, enthaltend als Komponente A ein Polymer erhalten mit Methylmethacrylat als Monomer b).

8. Dosierungsformen nach einem der Ansprüche 1 bis 7 , enthaltend als Komponente A der äusseren Überzugsschicht ein Polymer aus 43 bis 47 Gew.- %, bezogen auf das Gesamtgewicht der zur Polymerisation eingesetzten Monomere, N,N-Diethylaminoethylmethacrylat a), und 53 bis 57 Gew.- %, bezogen auf das Gesamtgewicht der zur Polymerisation eingesetzten Monomere, wenigstens einer Verbindung b), insbesondere Methylmethacrylat,

9. Dosierungsformen nach einem der Ansprüche 1 bis 8, enthaltend in der äusseren Überzugsschicht als Komponente B ein oder mehrere Antioxidantien.

10. Dosierungsformen nach einem der Ansprüche 1 bis 9, enthaltend in der äusseren Überzugsschicht als Komponente B ein oder mehrere Antioxidantien aus der Gruppe bestehend aus phenolischen Antioxidantien, thiolischen Antioxidantien, basischen Aminosäuren, Alkalicarbonate und Alkalibicarbonaten .

11. Dosierungsformen nach einem der Ansprüche 1 bis 10, enthaltend in der äusseren Überzugsschicht als Komponente C einen oder mehrere Weichmacher.

12. Dosierungsformen nach einem der Ansprüche 1 bis 11, enthaltend in der äusseren Überzugsschicht ii) als Komponenten C) Weichmacher aus der Gruppe bestehend aus Tributylcitrat, Acetyltributylcitrat, Acetyltriethylcitrat, Triacetin, Triethylcitrat, Diethylsebacat und Dibutylsebacat.

13. Dosierungsformen nach einem der Ansprüche 1 bis 12, enthaltend in der äusseren Überzugsschicht als Komponenten D weitere pharmazeutische Hilfsstoffe Aromastoffe, geschmacksverbessernde Stoffe, Süßungsmittel , Gleitmittel, Netzmittel, Trennmittel, Antiklebemittel, Stabilisatoren, Porenbildner, Neutralisierungsmittel, Glanzmittel, Farbstoffe, Pigmente, Desinfektions- oder Konservierungsmittel, Verdickungsmittel.

14. Dosierungsformen nach einem der Ansprüche 1 bis 13, enthaltend eine innere Überzugsschicht mit Schichtdicken von 1 bis 50 µm.

15. Dosierungsformen nach einem der Ansprüche 1 bis 14, enthaltend eine innere Überzugsschicht mit Schichtdicken von 2 bis 25 µm.

16. Dosierungsformen nach einem der Ansprüche 1 bis 15, enthaltend eine innere Überzugsschicht mit Schichtdicken von 5 bis 15 µm.

17. Dosierungsformen nach einem der Ansprüche 1 bis 16, enthaltend eine äußere Überzugsschicht mit Schichtdicken von 5 bis 200 µm.

18. Dosierungsformen nach einem der Ansprüche 1 bis 17, enthaltend eine äußere Überzugsschicht mit Schichtdicken von 10 bis 150 µm.

19. Dosierungsformen nach einem der Ansprüche 1 bis 18, enthaltend eine äußere Überzugsschicht mit Schichtdicken von 20 bis100µm.

## Claims

1. A dosage form provided with protective coatings, in which a core comprising at least one acidic active ingredient is provided with at least one internal and one external coating layer, where the outer layer comprises, as component A, a cationic polymer which is an emulsion polymer of N,N-diethylaminoethyl methacrylate and further monomers and the internal coating layer consists of a neutral water-soluble polymer and optionally pigments and further pharmaceutically customary excipients and use is made as neutral polymers of the internal coating layer, of polyvinyl alcohols, polyalkylene glycol - polyvinyl alcohol graft copolymers, polyvinylpyrrolidones, vinylpyrrolidone - vinyl acetate copolymers, alkylated and hydroxyalkylated celluloses or starches or mixtures of such polymers.

2. The dosage form according to claim 1, comprising, as acidic active ingredients, active ingredients with free carboxyl, sulfonic acid or phosphonic acid groups, acidic hydroxyl groups, acidic N-H groups or acidic C-H groups or mixtures of such active ingredients.

3. The dosage form according to claim 1 or 2, comprising acidic active ingredients with a pKa value between 6.5 and 0.5.

4. The dosage form according to any one of claims 1 to 3, comprising acidic active ingredients with a pKa value between 5.0 and 1.0.

5. The dosage form according to any one of claims 1 to 4, comprising, as neutral polymers of the internal coating layer, polyvinyl alcohols, polyalkylene glycol - polyvinyl alcohol graft copolymers or mixtures thereof.

6. The dosage form according to any one of claims 1 to 5, comprising, as component A of the outer coating layer, a polymer, obtained by free-radical polymerization, of
a) N,N-diethylaminoethyl methacrylate, and
b) at least one free-radically polymerizable compound selected from esters of α,β-ethylenically unsaturated mono- and dicarboxylic acids with C₁-C₈-alkanols,

7. The dosage form according to any one of claims 1 to 6, comprising, as component A, a polymer obtained with methyl methacrylate as monomer b).

8. The dosage form according to any one of claims 1 to 7, comprising, as component A of the outer coating layer, a polymer of 43 to 47% by weight, based on the total weight of the monomers used for the polymerization, of N,N-diethylaminoethyl methacrylate a), and 53 to 57% by weight, based on the total weight of the monomers used for the polymerization, of at least one compound b), in particular methyl methacrylate.

9. The dosage form according to any one of claims 1 to 8, comprising, in the outer coating layer as component B, one or more antioxidants.

10. The dosage form according to any one of claims 1 to 9, comprising, in the outer coating layer as component B, one or more antioxidants from the group consisting of phenolic antioxidants, thiolic antioxidants, basic amino acids, alkali metal carbonates and alkali metal bicarbonates.

11. The dosage form according to any one of claims 1 to 10, comprising, in the outer coating layer as component C, one or more plasticizers.

12. The dosage form according to any one of claims 1 to 11, comprising, in the outer coating layer ii) as components C), plasticizers from the group consisting of tributyl citrate, acetyltributyl citrate, acetyltriethyl citrate, triacetin, triethyl citrate, diethyl sebacate and dibutyl sebacate.

13. The dosage form according to any one of claims 1 to 12, comprising, in the outer coating layer as components D, further pharmaceutical excipients aroma substances, taste-improving substances, sweeteners, glidants, wetting agents, release agents, antiadhesives, stabilizers, pore formers, neutralizing agents, polishes, dyes, pigments, disinfectants or preservatives, thickeners.

14. The dosage form according to any one of claims 1 to 13, comprising an inner coating layer with layer thicknesses of from 1 to 50 µm.

15. The dosage form according to any one of claims 1 to 14, comprising an inner coating layer with layer thicknesses of from 2 to 25 µm.

16. The dosage form according to any one of claims 1 to 15, comprising an inner coating layer with layer thicknesses of from 5 to 15 µm.

17. The dosage form according to any one of claims 1 to 16, comprising an outer coating layer with layer thicknesses of from 5 to 200 µm.

18. The dosage form according to any one of claims 1 to 17, comprising an outer coating layer with layer thicknesses of from 10 to 150 µm.

19. The dosage form according to any one of claims 1 to 18, comprising an outer coating layer with layer thicknesses of from 20 to 100 µm.

## Revendications

1. Formes de dosage pourvues de revêtements de protection, dans lesquelles un noyau contenant au moins une substance active acide est pourvu d'au moins une couche de revêtement interne et une couche de revêtement externe, la couche externe contenant, comme composant A, un polymère cationique, qui représente un polymère en émulsion de méthacrylate de N,N-diéthylaminoéthyle et d'autres monomères et la couche de revêtement interne étant constituée par un polymère neutre soluble dans l'eau et éventuellement par des pigments et d'autres adjuvants pharmaceutiquement usuels et des poly(alcools vinyliques), des copolymères greffés de polyalkylèneglycol-poly(alcool vinylique), des polyvinylpyrrolidones, des copolymères de vinylpyrrolidone-acétate de vinyle, des celluloses ou des amidons alkylé(e)s et hydroxyalkylé(e)s ou des mélanges de ces polymères étant utilisés comme polymères neutres de la couche de revêtement interne.

2. Formes de dosage selon la revendication 1, contenant, comme substances actives acides, des substances actives présentant des groupes carboxyle, acide sulfonique ou acide phosphonique libres, des groupes hydroxyle acides, des groupes N-H acides ou des groupes C-H acides ou des mélanges de telles substances actives.

3. Formes de dosage selon la revendication 1 ou 2, contenant des substances actives acides présentant une valeur pKa entre 6,5 et 0,5.

4. Formes de dosage selon l'une quelconque des revendications 1 à 3, contenant des substances actives acides présentant une valeur pKa entre 5,0 et 1,0.

5. Formes de dosage selon l'une quelconque des revendications 1 à 4, contenant, comme polymères neutres de la couche de revêtement interne, des poly(alcools vinyliques), des copolymères greffés de polyalkylèneglycol-poly(alcool vinylique) ou des mélanges de ceux-ci.

6. Formes de dosage selon l'une quelconque des revendications 1 à 5, contenant comme composant A de la couche de revêtement externe un polymère obtenu par polymérisation par voie radicalaire de
a) méthacrylate de N,N-diéthylaminoéthyle, et
b) au moins un composé polymérisable par voie radicalaire, choisi parmi les esters d'acides monocarboxyliques et dicarboxyliques éthyléniquement α,ß-insaturés avec des C₁-C₈-alcanols.

7. Formes de dosage selon l'une quelconque des revendications 1 à 6, contenant comme composant A un polymère obtenu avec du méthacrylate de méthyle comme monomère b).

8. Formes de dosage selon l'une quelconque des revendications 1 à 7, contenant comme composant A de la couche de revêtement externe un polymère constitué par 43 à 47% en poids, par rapport au poids total des monomères utilisés pour la polymérisation, de méthacrylate de N,N-diéthylaminoéthyle a) et par 53 à 57% en poids, par rapport au poids total des monomères utilisés pour la polymérisation, d'au moins un composé b), en particulier le méthacrylate de méthyle.

9. Formes de dosage selon l'une quelconque des revendications 1 à 8, contenant dans la couche de revêtement externe, comme composant B, un ou plusieurs antioxydants.

10. Formes de dosage selon l'une quelconque des revendications 1 à 9, contenant dans la couche de revêtement externe, comme composant B, un ou plusieurs antioxydants du groupe constitué par les antioxydants phénoliques, les antioxydants thioliques, les acides aminés basiques, les carbonates de métal alcalin et les bicarbonates de métal alcalin.

11. Formes de dosage selon l'une quelconque des revendications 1 à 10, contenant dans la couche de revêtement externe, comme composant C, un ou plusieurs plastifiants.

12. Formes de dosage selon l'une quelconque des revendications 1 à 11, contenant dans la couche de revêtement externe ii), comme composants C, des plastifiants du groupe constitué par le citrate de tributyle, le citrate d'acétyltributyle, le citrate d'acétyltriéthyle, la triacétine, le citrate de triéthyle, le sébacate de diéthyle et le sébacate de dibutyle.

13. Formes de dosage selon l'une quelconque des revendications 1 à 12, contenant, dans la couche de revêtement externe, comme composants D, d'autres adjuvants pharmaceutiques, parfums, agents d'amélioration du goût, édulcorants, agents de glissement, agents mouillants, agents de démoulage, agents antiadhésifs, stabilisateurs, agents de formation de pores, agents de neutralisation, agents nacrés, colorants, pigments, agents de désinfection ou de conservation, épaississants.

14. Formes de dosage selon l'une quelconque des revendications 1 à 13, contenant une couche de revêtement interne présentant des épaisseurs de couche de 1 à 50 µm.

15. Formes de dosage selon l'une quelconque des revendications 1 à 14, contenant une couche de revêtement interne présentant des épaisseurs de couche de 2 à 25 µm.

16. Formes de dosage selon l'une quelconque des revendications 1 à 15, contenant une couche de revêtement interne présentant des épaisseurs de couche de 5 à 15 µm.

17. Formes de dosage selon l'une quelconque des revendications 1 à 16, contenant une couche de revêtement externe présentant des épaisseurs de couche de 5 à 200 µm.

18. Formes de dosage selon l'une quelconque des revendications 1 à 17, contenant une couche de revêtement externe présentant des épaisseurs de couche de 10 à 150 µm.

19. Formes de dosage selon l'une quelconque des revendications 1 à 18, contenant une couche de revêtement externe présentant des épaisseurs de couche de 20 à 100 µm.
